Europäisches Patentamt

European Patent Office

Office européen des brevets

(11) Veröffentlichungsnummer: **0 232 853**
**A2**

(12) **EUROPÄISCHE PATENTANMELDUNG**

(21) Anmeldenummer: 87101529.3

(22) Anmeldetag: 05.02.87

(51) Int. Cl.4: **B01J 8/08** , C02F 3/06 ,
C02F 3/28 , C12N 11/00 ,
C12M 1/00

(30) Priorität: 07.02.86 DE 3603792

(43) Veröffentlichungstag der Anmeldung:
19.08.87 Patentblatt 87/34

(84) Benannte Vertragsstaaten:
AT DE FR GB NL

(71) Anmelder: Caro, Thomas, Dipl.-Ing.
Georgstrasse 24
D-5100 Aachen(DE)

(72) Erfinder: Caro, Thomas, Dipl.-Ing.
Georgstrasse 24
D-5100 Aachen(DE)

(74) Vertreter: Werner, Hans-Karsten, Dr. et al
Deichmannhaus am Hauptbahnhof
D-5000 Köln 1(DE)

(54) **Mehrstufiger reaktor zur Stoffumwandlung mittels Katalysatoren und Verfahren zu dessen Durchführung.**

(57) Bei einem mehrstufigen Reaktor zur Stoffumwandlung mittels Katalysatoren wird der Rohstoff durch einen schrägen Zulauf im untersten Abschnitt eines Reaktors mit trichterförmigem Boden zugeführt, durch die tangentiale Zufuhr von Flüssigkeit der Reaktorinhalt zur Rotation und in innigen Kontakt mit den Katalysatoren gebracht, die Flüssigkeit durch Überströmkanäle in die nächsten Stufen geleitet, deren Böden ebenfalls trichterförmig ausgelegt sind, um dort der weiteren Reaktion unterworfen zu werden und hinter der letzten Stufe am oberen Ende des Reaktors abgezogen zu werden.

Fig. 3

EP 0 232 853 A2

## Mehrstufiger Reaktor zur Stoffumwandlung mittels Katalysatoren und Verfahren zu dessen Durchführung

Die Erfindung betrifft einen neuen Reaktor zur Stoffumwandlung mittels Katalysatoren und ein neues mehrstufiges Verfahren zur Durchführung desselben.

Mehrstufige Verfahren zur Stoffumwandlung mittels Katalysatoren sind schon seit langem bekannt. Für solche Prozesse werden in der Regel mehrere Reaktoren hintereinandergeschaltet. Mehrstufige Verfahren lassen sich jedoch auch in einem einzigen Reaktor durchführen. Als Alternative zum Hintereinanderschalten von mehreren Apparaten ist beispielsweise der Siebboden-Reaktor anzusehen - (Nesemann Chem. Ztg. 98, 1974, Seite 526; Rehm. Chem.-Ing.-Tech. 42, 1970, Seite 584; DE-PS 19 11 038; 16 42 594; 16 42 653). Eine abgewandelte Form des Siebboden-Reaktors ist der dreistufige Rührfermenter nach Brauer & Schmidt (Brauer CAV, 1974, Seite 73 ff).

Aus der DE-PS 16 42 594 ist ein Turmreaktor bekannt, der durch Trennorgane in mehrere vertikal übereinander angeordnete Kammern aufgeteilt ist. Die Fermentationsflüssigkeit wird einer unteren Kammer zugeführt, und die Produkte werden aus einer oberen Kammer abgezogen. Die Trennorgane sind so ausgebildet, daß sie den Gasstrom zwar durchlassen, jedoch begrenzen, so daß im oberen Teil jeder Kammer eine Gastasche gebildet wird. Hierdurch wird der Flüssigkeitsstrom zwischen den einzelnen Kammern unterbrochen und die Fermentationsbrühe mittels des Gasstromes durch die Gastaschen mitgerissen. Als Trennorgane werden perforierte Platten eingesetzt.

Der Reaktor eignet sich nur für aerobe Prozesse. Die zugeführte Luft wird von unten nach oben durch den gesamten Reaktor geführt. Durch den starken Luftstrom und die daraus resultierende intensive Verwirbelung der Fermentationslösung werden die Organismen aus der letzten Stufe ausgewaschen. Der starke Gasstrom bewirkt zudem, daß die Organismen über die Trennorgane hinweg in die nächste Stufe überführt werden.

Aus der FR-PS 78 23 978 ist ebenfalls ein Reaktor bekannt, der durch gelochte Platten in Kammern aufgeteilt ist. Zusätzlich sind die einzelnen Stufen durch Überlaufrohre miteinander verbunden. Enzym und Substrat werden im Gegenstrom geführt, wobei die Enzyme über die Überlaufrohre von einer Stufe zur anderen gelangen.

Aus der DE-PS 12 67 674 ist eine Vorrichtung zur Kohlenoxidhydrierung bekannt, die aus einem senkrechten, zylindrischen Gefäß besteht. Unterteilt ist dieses durch einen oder mehrere konisch nach unten zulaufende und mit einer an der Spitze gelegenen Öffnung versehene Zwischenböden. Der Reaktor kann nur als Blasensäule betrieben werden. Das Gas steigt in Form von feinen Blasen durch die Flüssigkeit aufwärts, wobei es sich zu Kohlenwasserstoffen umsetzt. Für immobilisierte Enzyme oder Mikroorganismen ist eine derartige Vorrichtung ungeeignet. Diese würden sich absetzen und in der untersten Stufe als großes Packbett wirken. Für die Verarbeitung flüssiger Medien ist die Reaktorkonstruktion ebenfalls nicht geeignet, da die Anwesenheit und die Mischwirkung der Gasblasen notwendige Voraussetzung für das Funktionieren des Reaktors sind.

Aus der DE-OS 33 26 879 ist schließlich ein Reaktor bekannt, der durch kegelförmige Doppeltrichter, bestehend aus einem Außentrichter und einem Innentrichter, unterteilt ist. Diese Trichter reichen nicht bis an die Reaktorwand. Die Doppeltrichter bilden einen Gasraum, durch den Gas abgeleitet werden soll. Die mitgerissenen Schlammteilchen und Biomasseteilchen sinken über einen Sedimentationsraum zurück. Der sich im Sedimentationsraum ansammelnde Schlamm fließt über die Kanten des Außentrichters in den darunterliegenden Raum zurück.

Die erwähnten Reaktoren lassen sich zum Teil sowohl in der chemischen Verfahrenstechnik als auch in der Bioverfahrenstechnik anwenden. Ein Überblick über die heute in der Biotechnik gebräuchlichen Bioreaktoren kann den einschlägigen Lehrbüchern entnommen werden (z.B. Rehm. "Industrielle Mikrobiologie", 2. Auflage, Berlin, Heidelberg, New York 1980; Präve u.a. "Handbuch der Biotechnologie", Wiesbaden 1982).

Die heute in der Biotechnik eingesetzten Submers-Fermenter arbeiten entweder diskontinuierlich oder kontinuierlich als gerührter oder hydraulisch durchmischter Behälter. Diese Bioreaktoren haben jedoch verschiedene Nachteile. So treten bei den diskontinuierlich betriebenen Bioreaktoren lange Totzeiten auf, die durch das Befüllen, Beimpfen, Anfahren, Ausräumen und Reinigen der Anlage bedingt sind. Außerdem fallen das Produkt und die Restmassen nicht kontinuierlich, sondern stoßweise an. Das führt wiederum zu erhöhten Betriebskosten, da die anfallenden Stoffe vor der Weiterverarbeitung zunächst zwischengelagert werden müssen.

Die kontinuierlich betriebenen Reaktoren geben hingegen bei homogener Vermischung des Inhaltes immer einen Teil des ursprünglich eingesetzten Rohstoffes unverwertet ab. Das heißt, mit solchen Reaktoren werden nicht die optimale Produktivität und Ausbeute erreicht.

Höhere Produktivität und bessere Ausbeuten bei geringeren Apparatedimensionen lassen sich mit heterogenen Reaktoren erreichen. Solche Reaktoren zeichnen sich dadurch aus, daß sie kontinuierlich betrieben werden können und die Substratumwandlung während des Durchlaufes des Behälters stetig zunimmt. In den verschiedenen Höhen des Apparates herrschen verschiedene Produktkonzentrationen. Die maximale Produktkonzentration liegt am Abzug vor, was die Aufarbeitung verbilligt. Ein weiterer Vorteil dieser Fahrweise liegt in der großen Umsatzgeschwindigkeit, die bei dem Einsatz von Mischkulturen (z.B. bei der Abwasserreinigung) in den einzelnen Abbauphasen durch eine genau adaptierte Mikroorganismen-Population hervorgerufen wird. Bei Reaktoren für heterogene Stoffumwandlungen treten jedoch heute noch Schwierigkeiten bei der Stromführung des Substrates, der Durchmischung und der Belastung der Biomasse auf.

In der Verfahrenstechnik werden heute auch zunehmend Mikroorganismen oder Enzyme als Katalysatoren eingesetzt. Diese Biokatalysatoren können entweder in Suspension oder in immobilisierter Form vorliegen. Insbesondere in der Abwassertechnologie ist es zur Zeit gebräuchlich, den anaeroben Schlamm als Suspension im Medium einzusetzen und die Biomasse in einem separaten Anlagenteil aus dem Produkt abzutrennen (Kontaktverfahren). Dabei entstehen oft Schwierigkeiten, weil die Organismen nur schlecht sedimentieren oder die Filter verstopft werden.

Enzyme werden heute meist in löslicher Form den Substraten zugegeben. Bei einer derartigen Verfahrensweise gehen die Enzyme verloren, so daß man sehr sparsam dosieren muß. Durch verschiedene Methoden ist es inzwischen möglich geworden, Enzyme und Mikroorganismen zu immobilisieren.

Dadurch verbessern sich die Sedimentationseigenschaften. Die Mikroorganismen können im System gehalten werden, ohne aufwendige Abscheidevorrichtungen einsetzen zu müssen. Immobilisierte Enzyme können so lange verwendet werden, bis sie ihre katalytischen Eigenschaften weitgehend verloren haben und ihre Restaktivität unwirtschaftlich wird (vgl. Chibata, "Immobilized Enzymes", J. Wiley & Sons, 1978, Seite 151). Durch die Immobilisierung von Biokatalysatoren ergeben sich ferner eine Reihe von weiteren Vorteilen bei der Konstruktion und dem Betrieb von Reaktoren. Hierzu zählen vor allem die höhere Umsatzrate durch die Anwendung des Prinzips des Rohrreaktors für heterogene Stoffumwandlungen, die höhere Produktivität, der Wegfall von Abtrenneinrichtungen, geringere Behälterdimensionen, die optimale Organismenadaptation durch heterogenen Substratabbau und die Ausnutzung der eingesetzten Enzyme bis zu ihrer Unwirtschaftlichkeit. Diese Faktoren zusammengenommen stehen für geringere Anschaffungs-und Betriebskosten.

Bei den industriellen Anwendern besteht daher eine deutliche Bereitschaft, in Zukunft vermehrt immobilisierte Mikroorganismen und Enzyme einzusetzen. Ein Rohrreaktor für heterogene Stoffumwandlungen, der auf die Anforderungen der immobilisierten Biokatalysatoren ausgelegt ist und keine besonderen Anforderungen an das Substrat stellt - (also auch so schwierige Flüssigkeiten wie kommunale Abwässer bewältigen kann), wird auf dem Markt zur Zeit nicht angeboten.

Die vorliegende Erfindung hat sich demgemäß die Aufgabe gestellt, einen mehrstufigen Reaktor für heterogene Stoffumwandlungen mittels Katalysatoren mit trichterförmigen Böden, einem Zulauf und einem Ablauf zu entwickeln.

Diese Aufgabe wird dadurch gelöst, daß

a) der Reaktor (1) durch Böden, die als Einfachtrichter ausgebildet sind, unterteilt ist,

b) die Böden Überströmkanäle (6) aufweisen,

c) der Zulauf im untersten Abschnitt des Reaktors so ausgelegt ist, daß die Flüssigkeit tangential zugeführt wird, und

d) in jeder Stufe ein Einfüllstutzen vorhanden ist.

Die Böden können umgekehrt trichterförmig ausgelegt sein, so daß sich in der Trichterspitze das entstehende Gas sammeln kann. In diesem Fall befindet sich in der Trichterspitze eine Gasabführung, die mit jeder Stufe über ein Schwimmerventil verbunden ist. Am oberen Ende der Gasableitung kann außerdem ein Schaumabscheider angebracht sein.

Der erfindungsgemäße Reaktor kann mit Hilfe von Katalysatoren, insbesondere von immobilisierten Mikroorganismen und/oder Enzymen, flüssige Substrate, insbesondere organisch hochbelastete Abwässer, kontinuierlich umsetzen. In einem heterogenen, mehrstufigen Turmreaktor soll durch den Einsatz von an Trägermaterial angebundenen Katalysatoren, insbesondere Mikroorganismen und Enzyme, soviel aktive Biomasse im Reaktor gehalten werden, daß schon nach einmaligem Durchlauf der Stufen eine weitestgehende Umsetzung des eingesetzten Rohmaterials stattgefunden hat. Hierdurch wird erreicht, daß die Apparateabmessungen gegenüber den bisher üblichen erheblich reduziert werden können. Infolgedessen wird die Rentabilitätsgrenze, z.B. bei der Abwasserreinigung, so weit herabgesetzt, daß der erfindungsgemäße Reaktor als Kompaktanlage für kleinere Betriebe verwendbar ist.

In einer weiteren Variante enthält der Reaktor eine Fördereinrichtung für die Führung der Katalysatoren im Gegenstrom zum Substrat, das durch Überströmkanäle in den Böden von unten nach oben fließt.

Die Erfindung betrifft ferner ein mehrstufiges Verfahren zur Stoffumwandlung mittels Katalysatoren in einem Reaktor, dadurch gekennzeichnet, daß

a) der Rohstoff durch einen schrägen Zulauf (2) im untersten Abschnitt des Reaktors (1) zugeführt wird,

b) durch die tangentiale Zufuhr von Flüssigkeit der Reaktorinhalt zur Rotation und in innigen Kontakt mit den Katalysatoren gebracht wird,

c) die Flüssigkeit durch Überströmkanäle - (6) in die nächsten Stufen gelangt,

d) dort der weiteren Reaktion unterworfen wird und

e) hinter der letzten Stufe am oberen Ende des Reaktors das Produkt abgezogen wird.

Als Katalysatoren können Enzyme oder Mikroorganismen eingesetzt werden. Im letzteren Fall eignet sich das erfindungsgemäße Verfahren insbesondere zur biologischen Abwasserreinigung. Hierfür werden Reaktoren mit umgekehrt trichterförmigen Böden bevorzugt. Dabei kann in einer oder mehreren Stufen zunächst eine Hydrolyse durchgeführt werden. Daran schließen sich in einer oder mehreren Stufen eine Acidogenese, eine Acetogenese und eine Methanogenese an. Hinter der letzten Stufe am oberen Ende des Reaktors kann dann das gereinigte Wasser abgezogen werden. Ein derartiges Verfahren eignet sich insbesondere zur Denitrifikation und Nitrifikation von Abwässern sowie zur Reinigung von Abwässern mit Metallbelastungen.

Mit dem erfindungsgemäßen Verfahren können auch Enzyme hergestellt werden. Ebenso lassen sich pharmazeutische, kosmetische und chemische Produkte, wie Ethanol, Butanol oder Aceton, erzeugen. Ferner eignet sich das erfindungsgemäße Verfahren zur Flüssigzuckerherstellung, Verzuckerung von Stärke, zum Abbau von in Abwässern gelösten Fetten, zur Verzuckerung von Milch und zur Klärung von Frucht-und Gemüsesäften. Als Katalysatoren können hierbei auch immobilisierte Enzyme eingesetzt werden. Darüber hinaus lassen sich aber ebenso Mikroorganismen und Enzyme gemeinsam in immobilisierter bzw. nicht-immobilisierter Form verwenden.

In einer weiteren Variante des erfindungsgemäßen Verfahrens können die Katalysatoren im Gegenstrom mittels einer Fördereinrichtung von oben nach unten durch die einzelnen Stufen geführt werden, während das Substrat über Überströmkanäle in den trichterförmigen Böden von unten nach oben durch die Stufen fließt.

Bei dem Substrat kann es sich beispielsweise um eine Saccharoselösung und bei dem Katalysator um ein Enzym (zum Beispiel Invertase zur Herstellung von Flüssigzucker) handeln. Das Substrat gelangt so stets in Bereiche mit höherer Enzymaktivität, was eine gute Umsatzrate erwarten läßt. Unwirtschaftlich gewordene Enzyme werden unten abgezogen und oben durch die entsprechende Menge frischer Enzyme ersetzt. Die Begrenzung der Schichthöhe erfolgt durch im Reaktor eingebaute Trennböden. Die Enzymmasse wird durch eine Fördereinrichtung gegen den Substratstrom bewegt, wodurch ein Aktivitätsgradient im Reaktor entsteht. Unten trifft frisches Substrat auf geschwächtes Enzym, welches diesen Nährstoff noch teilweise umsetzen kann.

Das frische und noch voll aktive Enzym trifft danach im oberen Behälter auf bereits weitgehend umgesetztes Substrat. In diesem Behälterbereich wird demzufolge die restliche Katalysierung durchgeführt, die von den weniger aktiven Enzymen nicht mehr vollzogen werden könnte. Durch eine derartige Verfahrensführung ist es möglich geworden, auf Dauer hohe Umsatzraten bei sparsamstem Enzymverbrauch zu erzielen.

Im folgenden werden der erfindungsgemäße Reaktor und das erfindungsgemäße Verfahren unter Bezugnahme auf die Zeichnungen näher erläutert:

Figur 1 zeigt das Prinzip eines Reaktors für die heterogene Produktbildung mit einem Zulauf und Abzug.

Figur 2 zeigt die Abhängigkeit zwischen der Höhe des Reaktors und der Umsatzrate.

Figur 3 zeigt das Konstruktionsprinzip einer vierstufigen Variante des erfindungsgemäßen Reaktors.

Figur 4 zeigt eine weitere Variante des erfindungsgemäßen Reaktors, bei der eingesetzter Rohstoff und Katalysator im Gegenstrom geführt werden.

Figur 5 zeigt einen Überströmkanal, wie er in den erfindungsgemäßen Reaktoren eingesetzt wird.

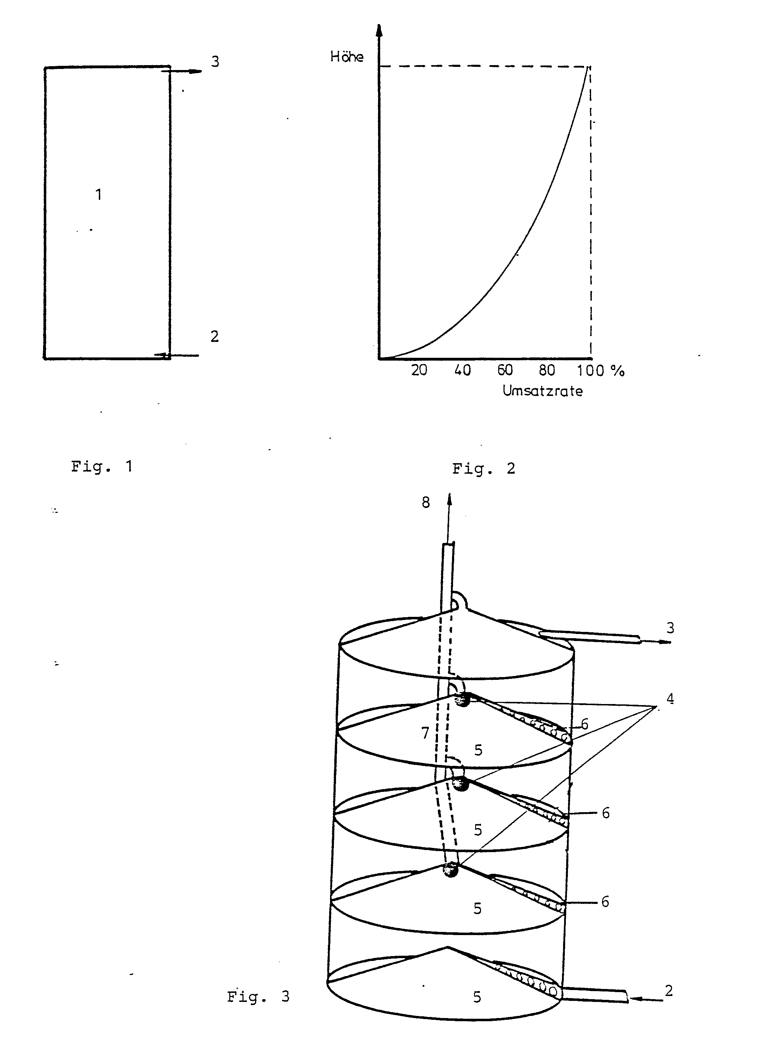

Aus Fig. 1 und 2 ist ersichtlich, daß mit zunehmender Höhe des Reaktors 1 die Produktkonzentration zunimmt. Über den Stutzen 2 wird das Substrat zugeführt, während man über den Stutzen 3 das Produkt, z.B. das gereinigte Abwasser, aberntet. Die maximale Produktkonzentration liegt am Abzug vor.

Der in Fig. 3 dargestellte Reaktor kann kontinuierlich und heterogen betrieben werden. Über den Stutzen 2 wird das Substrat zugeführt, während man über den Stutzen 3 das Produkt, z.B. das gereinigte Abwasser, aberntet. Durch spezielle Einbauten wird der Behälter in einzelne Stufen unterteilt. In diesen Stufen, die von außen durch Einfüllstutzen zugänglich sind, wirken die Katalysatoren, z.B. immobilisierte Mikroorganismen oder Enzyme, auf das durch Überströmkanäle 6 von unten zu fließende Substrat. Durch eine tangentiale Zuführung des Substrates in die einzelnen Stufen wird eine Rotationsbewegung erzeugt, so daß die Katalysatoren und die umzuwandelnden Stoffe durchmischt werden können.

Die Böden 5 der einzelnen Stufen sind umgekehrt trichterförmig ausgelegt, so daß eventuell entstehende Gase sich in der Mitte sammeln und durch das Zentralrohr 7 abgeleitet werden können. Der Einlauf des Ableitungsrohrs ist mit einem Schwimmerventil 4 versehen, das den Flüssigkeitsstand im Trichter regelt und die Trennung von Flüssigkeit und Gas bewirkt. Bei steigender Gasmenge im Trichter sinkt der Flüssigkeitsspiegel ab. Hierdurch wird das Ventil im Ableitungsrohr geöffnet und das Gas kann durch das Zentralrohr 7 nach oben abströmen. Bei dieser Konstruktion kann das Produktgas in einem einzigen gemeinsamen Ableitungsrohr gesammelt und über das Abgasrohr 8 abgeführt werden.

Bei Prozessen mit Gasproduktion sind die Böden umgekehrt trichterförmig angebracht. Die Gasabfuhr erfolgt dann über ein Schwimmerventil. Bei Prozessen ohne oder mit nur geringfügiger Gasproduktion können die Böden auch trichterförmig angeordnet sein.

Die Unterteilung des Reaktors in einzelne Stufen gestattet den Betrieb mit Mischkulturen, also mit einem breiten Spektrum von Mikroorganismen, die in Symbiose leben. Hierbei reichern sich in jeder Stufe jeweils an das zuströmende Substrat adaptierte Populationen an. In den unteren Reaktorstufen siedeln sich aufgrund der starken Belastung des Abwassers überwiegend hydrolisierende Organismen an. In der Mitte überwiegen die Säurebildner und in den oberen Stufen sind hauptsächlich Methanbildner zu finden. Die Populationen in den jeweiligen Stufen sind somit optimal an das jeweils angebotene Substrat angepaßt. Hierdurch wird eine bedeutende Beschleunigung des Umsatzvorganges bewirkt. Das bedeutet, daß bei gleicher Abbaurate der Abwasserschadstoffe ein wesentlich kleinerer Reaktionsbehälter notwendig ist. So kann beispielsweise bei der Ethanolherstellung die Behältergröße um etwa 90% gegenüber herkömmlichen Apparaten reduziert werden. Bei der Biogasherstellung wird die Anzahl der Stufen durch die Höhe der Gasproduktion bestimmt. Es können also auch mehrere Stufen, z.B. für die Methanogenese, dienen.

Im Falle der Verwendung von immobilisierten Enzymen oder Mikroorganismen werden die einzelnen Stufen mit Füllkörpern bestückt, die den Organismen eine Aufwuchsfläche bieten. Dadurch werden sie an diese Träger immobilisiert. Als Füllkörper sind insbesondere Sand, Blähgestein und andere diverse künstliche Füllkörper geeignet. Die überschüssige Biomasse kann hierbei aus jeder Stufe separat über die Einfüllstutzen abgezogen werden. Bei selbstständig flockenden Organismen kann auf Füllkörper verzichtet werden.

Bei Verwendung von Mikroorganismen sollen zur optimalen Auslastung des Reaktors in den verschiedenen Stufen jeweils die Organismen angesiedelt werden, die an das zufließende Substrat adaptiert sind. Im Fermenter liegt also neben einer heterogenen Substratverteilung auch eine heterogene Organismenverteilung vor. Auf diese Art werden die Produktivität bzw. bei der Abwasserreinigung der Schadstoffabbau gesteigert.

Die Regulation des Wachstums der in den einzelnen Stufen vorhandenen Mikroorganismen kann automatisch durchgeführt werden, indem nicht immobilisierte Organismen mit dem Produkt ausgeschwemmt werden. Man kann aber eine Regulation auch erreichen, indem überschüssige Festmasse aus jeder einzelnen Stufe abgezogen wird.

Der Anwendungsbereich des in Fig. 3 gezeigten Reaktors soll vor allem bei der Abwasserreinigung liegen. Ein derartiger Reaktor eignet sich nicht nur für die Behandlung großer Abwassermengen, sondern auch für in Kleinbetrieben oder im privaten Bereich anfallende geringere Mengen. Die kompakte Apparatur ermöglicht es, Abwässer am Entstehungsort unter Gewinnung von Biogas zu entsorgen. Es ist vorstellbar, daß bei etwas abseits liegenden Einzelhäusern solche Kleinkläranlagen installiert werden, um das anfallende Abwasser anaerob abzubauen. Daneben wäre das gewonnene Biogas als Energiequelle verwertbar. In Verbindung mit einer Hydrolysestufe für Feststoffe können sogar feste organische Abfälle (z.B. Papier, Pappe, Essensreste, landwirtschaftliche Abfälle) zu Biogas verarbeitet werden. Gegebenenfalls wird für die Hydrolyse ein zusätzlicher Reaktor eingesetzt.

Der erfindungsgemäße Reaktor zeichnet sich insbesondere durch die maximale Produktkonzentration im Auslauf und die damit erleichterte Aufarbeitung aus.

Gleichzeitig wird eine optimale Substratverwertung erreicht. Durch den Einsatz von immobilisierter Biomasse fallen im mehrstufigen Verfahren Abtrennung und Rückführung der Biomasse weg. Bei Einsatz von Mischkulturen liegen in jeder Stufe optimal adaptierte Populationen vor. Bei der Verwendung als Enzymreaktor erreicht man außerdem eine weitgehende Enzymauswertung bis zu geringen Restaktivitäten. In jeder Stufe bestehen Zugriffsmöglichkeiten für die Erhaltung der optimalen Zelldichte, zur Reinigung und Beseitigung von Verstopfungen. Durch die spezielle Reaktorkonstruktion können hohe Konzentrationen an Biomasse erreicht werden, ohne daß Aktivitätsverluste durch zu dichte Packung auftreten. Die multifunktionale Modulbauweise hat außerdem den Vorteil, daß der Reaktor an verschiedene Fermentationsbedingungen auf einfachste Weise angepaßt werden kann.

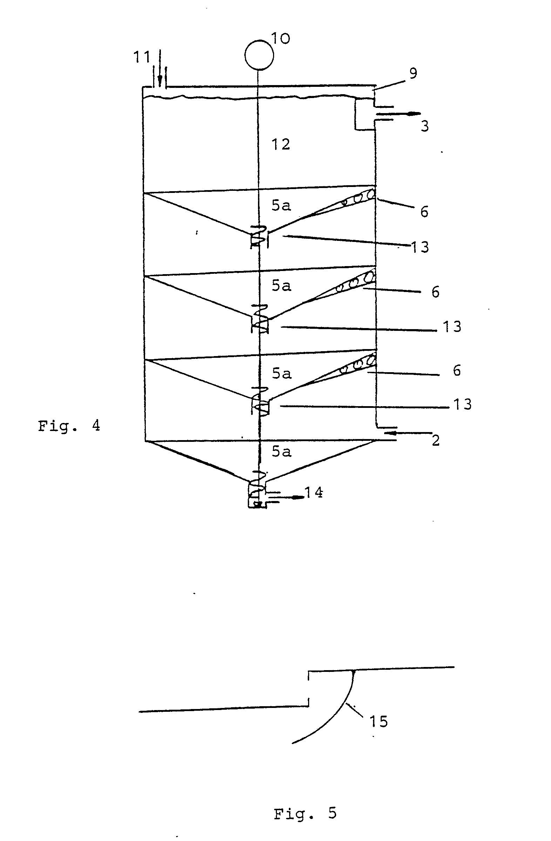

In Fig. 4 wird eine weitere Variante des erfindungsgemäßen Reaktors dargestellt, die sich besonders für die Verwendung als Enzymreaktor eignet. Auch dieser Reaktor kann kontinuierlich betrieben werden. Dabei werden Substrat und immobilisiertes Enzym im Gegenstrom geführt. Unwirtschaftlich gewordene Enzyme werden unten über den Stutzen 14 abgezogen und oben über den Stutzen 11 durch die entsprechende Menge frischer Enzyme ersetzt. Die Begrenzung der Schichthöhe erfolgt durch die eingebauten trichterförmigen Trennböden 5. Die Enzymmasse wird durch eine Fördereinrichtung, z.B. eine langsam laufende Schnecke 13, gegen den Substratstrom bewegt, wodurch ein Aktivitätsgradient im Reaktor entsteht. Die Fördereinrichtung wird über einen Motor 10 und eine Welle 12 angetrieben. Das Substrat gelangt über Überströmkanäle 6 in die jeweils nächste Stufe des Reaktors. Über einen Überlauf 9 und den Stutzen 3 wird das Produkt oben abgezogen. Über den Stutzen 2 wird frisches Substrat zugesetzt.

Das Substrat gelangt so stets in Bereiche mit höherer Enzymaktivität, was eine gute Umsatzrate bewirkt. Unten trifft das frische Substrat auf ein geschwächtes Enzym, welches an diesem Nährstoff noch befriedigende Umsätze bewirken kann. Das frische und noch voll aktive Enzym trifft am oberen Behälter auf das bereits weitgehend umgesetzte Substrat. Hier wird die restliche Katalysierung durchgeführt, die von weniger aktiven Enzymen nicht mehr vollzogen werden kann. Auf diese Art ist es möglich, auf Dauer hohe Umsatzraten bei sparsamstem Enzymverbrauch zu erzielen.

In Fig. 5 ist ein Überströmkanal dargestellt, wie er in den in Fig. 3 und 4 abgebildeten Reaktoren verwendet wird. Dieser ist so mit einem Gleitblech 15 abgedeckt, daß entstehendes Gas nicht in die nächste Stufe gelangt, sondern sich in der Trichtermitte der einzelnen Böden sammelt und von dort über das Zentralrohr nach oben abgeführt wird.

Die folgenden Beispiele sollen dazu dienen, das Reaktorprinzip zu erläutern und die gewünschten Auswirkungen der Konstruktion und Fahrweise der Anlage zu verdeutlichen. Die Werte sind als relativ anzusehen, da sie stark vom jeweiligen Betriebszustand und vom Substrat abhängen.

Es wurde ein Versuchsreaktor erstellt, der nach dem in Fig. 3 dargestellten Prinzip aufgebaut ist. Er besteht aus einer senkrechten, länglichen Röhre, die von umgekehrt trichterförmigen Böden in 5 Stufen unterteilt ist. Das entstehende Biogas wird aus jeder Stufe abgezogen, während die Flüssigkeit durch einen Überströmkanal zur nächsten Stufe gelangt. Der Reaktor wird von unten nach oben durchströmt.

Entsprechend wurde die Numerierung der Stufen vorgenommen (1 bis 5). Zur Immobilisation sind die Mikroorganismen hier an feine Kristalle angebunden.

Beispiel 1

Heterogener Abbau der Abwasserbelastung

Als Maß für die Belastung des Abwassers dient der CSB-Wert. Im Zulauf     8000 mg/ltr
In stufe 1  3420 mg/ltr
In Stufe 2  1760 mg/ltr
In Stufe 3  1308 mg/ltr
In Stufe 4  1150 mg/ltr
In Stufe 5  1182 mg/ltr

Beispiel 2

Heterogene Fermentationsbedingungen

In den einzelnen Stufen kann zum Beispiel ein pH-Gradient erzielt werden. In Stufe 1  pH 6,64
In Stufe 2  pH 6,92
In Stufe 4  pH 6,98
In Stufe 5  pH 7,07

Beispiel 3

Heterogene Organismenverteilung im Reaktor

Um einen optimalen Umsatz zu erreichen, sollten in den unteren Stufen die acetogenen Bakterien stärker vertreten sein als in den oberen Stufen. Als Maß für deren Aktivität können einige Säurekonzentrationen dienen.
Essigsäure:
In Stufe 1  348 mg/ltr
In Stufe 2  129 mg/ltr
In Stufe 3   24 mg/ltr
In Stufe 4    4 mg/ltr
In Stufe 5    2 mg/ltr
Milchsäure:
In Stufe 1    2 mg/ltr
In Stufe 5    nicht nachweisbar

**Ansprüche**

1. Mehrstufiger Reaktor für heterogene Stoffumwandlungen mittels Katalysatoren mit trichterförmigen Böden (5), einem Zulauf (2) und einem Ablauf (3), dadurch gekennzeichnet, daß

a) der Reaktor (1) durch Böden, die als Einfachtrichter ausgebildet sind, unterteilt ist,

b) die Böden Überströmkanäle (6) aufweisen,

c) der Zulauf im untersten Abschnitt des Reaktors so ausgelegt ist, daß die Flüssigkeit tangential zugeführt wird, und

d) in jeder Stufe ein Einfüllstutzen vorhanden ist.

2. Reaktor nach Anspruch 1, dadurch gekennzeichnet, daß die Böden (5) umgekehrt trichterförmig ausgelegt sind, sich in der Mitte eine Gasabführung (7) befindet, die mit jeder Stufe über ein Schwimmerventil (4) verbunden ist, und gegebenenfalls am oberen Ende der Gasableitung ein Schaumabscheider angebracht ist.

3. Reaktor nach Anspruch 1, dadurch gekennzeichnet, daß er eine Fördereinrichtung (13) für die Führung der Katalysatoren im Gegenstrom zum Substrat und in den trichterförmigen Böden (5a) Überströmkanäle (6) enthält, durch die Substrat von unten nach oben fließt.

4. Mehrstufiges Verfahren zur Stoffumwandlung mittels Katalysatoren in einem Reaktor gemäß Ansprüchen 1 bis 3, dadurch gekennzeichnet, daß

a) der Rohstoff durch einen schrägen Zulauf (2) im untersten Abschnitt des Reaktors (1) zugeführt wird,

b) durch die tangentiale Zufuhr von Flüssigkeit der Reaktorinhalt zur Rotation und in innigen Kontakt mit den Katalysatoren gebracht wird,

c) die Flüssigkeit durch Überströmkanäle - (6) in die nächsten Stufen gelangt,

d) dort der weiteren Reaktion unterworfen wird und

e) hinter der letzten Stufe am oberen Ende des Reaktors das Produkt abgezogen wird.

5. Verfahren nach Anspruch 4, dadurch gekennzeichnet, daß die gebildeten Gase durch umgekehrt trichterförmige Böden (5) gesammelt und über ein zentrales Ableitungsrohr (7) abgeführt werden, das mit jeder Stufe über ein Schwimmerventil (4) verbunden ist.

6. Verfahren nach Anspruch 4, dadurch gekennzeichnet, daß die Katalysatoren im Gegenstrom mittels einer Fördereinrichtung (13) von oben nach unten durch die einzelnen Stufen geführt werden, während das Substrat über Überströmkanäle (6) in den trichterförmigen Böden (5a) von unten nach oben durch die Stufen fließt.

7. Verfahren nach Ansprüchen 4 bis 6, dadurch gekennzeichnet, daß als Katalysatoren Mikroorganismen oder Enzyme eingesetzt werden.

8. Verfahren nach Anspruch 6, dadurch gekennzeichnet, daß immobilisierte Enzyme allein oder zusammen mit Mikroorganismen eingesetzt werden, wobei die Mikroorganismen an Trägern wachsen, die aufgrund ihrer Größe nicht durch die Fördereinrichtung in die anderen Stufen gelangen können, während im Reaktor ein Aktivitätsgradient ausgebildet wird, indem man die Enzymmasse durch eine Fördereinrichtung (13) gegen den Substratstrom von oben nach unten bewegt und dabei verbrauchte Enzyme unten abgezogen sowie oben durch die entsprechende Menge frischer Enzyme ersetzt werden.

9. Mehrstufiges Verfahren nach Ansprüchen 4 oder 5 zur Biogasherstellung und/oder zur biologischen Abwasserreinigung, insbesondere zur Denitrifikation und Nitrifikation, sowie zur Reinigung von Abwässern mit Metallbelastungen.

10. Verfahren zur biologischen Abwasserreinigung nach Ansprüchen 4 oder 5, dadurch gekennzeichnet, daß in einer oder mehreren Stufen Hydrolysen, Acidogenesen und Methanogenesen durchgeführt werden und hinter der letzten Stufe am oberen Ende des Reaktors das gereinigte Wasser abgezogen wird.

11. Verfahren nach Ansprüchen 4 bis 7 zur Herstellung von Enzymen, pharmazeutischen, kosmetischen und chemischen Produkten sowie zur Flüssigzuckerherstellung, Verzuckerung von Stärke, Abbau von in Abwässern gelösten Fetten, Verzuckerung von Milch und zur Klärung von Frucht-und Gemüsesäften.

12. Verwendung des Reaktors gemäß Ansprüchen 1 bis 3 für mikrobiologische und enzymkatalysierte Prozesse, dadurch gekennzeichnet, daß die Stufen mit geeigneten Füllkörpern, an denen Mikroorganismen und/oder Enzyme haften, bestückt sind.

13. Verwendung des Reaktors gemäß Ansprüchen 1 oder 2 zur Biogasherstellung und zur Reinigung von Abwässern, insbesondere zur Denitrifikation und Nitrifikation von Abwässern sowie zur Reinigung von Abwässern mit Metallbelastungen.

14. Verwendung des Reaktors gemäß Ansprüchen 1 bis 3 zur Herstellung von Enzymen, pharmazeutischen, kosmetischen und chemischen Produkten sowie zur Flüssigzuckerherstellung, Verzuckerung von Stärke, Abbau von in Abwässern gelösten Fetten, Verzuckerung von Milch und zur Klärung von Frucht-und Gemüsesäften mit Hilfe von immobilisierten Enzymen.

Fig. 1

Fig. 2

Fig. 3

Fig. 4

Fig. 5